# EUROPEAN PATENT APPLICATION

(11) **EP 3 904 778 A1**
(43) Date of publication of application: **03.11.2021**
(21) Application number: 19905239.0
(22) Date of filing: 13.11.2019
(51) Int. Cl.: F24F 11/64, F24F 11/65, F24F 11/61, F24F 11/38, F24F 11/52

(54) **METHOD FOR CONTROLLING AIR CONDITIONER PROVIDED WITH BIPOLAR ION GENERATION MODULE, AND AIR CONDITIONER**

(30) Priority: 27.12.2018 CN 201811613604
(71) Applicant: Qingdao Haier Air-Conditioning Electronic Co., Ltd, Qingdao, Shandong 266101 (CN); Haier Smart Home Co., Ltd., Qingdao, Shandong 266101 (CN)
(72) Inventor: ZHANG, Jiyi, Qingdao, Shandong 266101 (CN); DONG, Dezhi, Qingdao, Shandong 266101 (CN); WANG, Haimei, Qingdao, Shandong 266101 (CN)
(74) Representative: Byrne, Declan
(86) International application number: PCT/CN2019/117937
(87) International publication number: WO 2020/134670

(57) **Abstract**

Provided is a method for controlling an air conditioner provided with a bipolar ion generation module. The method comprises the following steps: an air conditioner controller determining whether a purification mode control signal for controlling a bipolar ion generation module to start operation is received; if the purification mode control signal is received, the air conditioner controller detecting whether an air conditioner is in a working state; if the air conditioner is in the working state, controlling the bipolar ion generation module to start working; and if the air conditioner is in a standby state, outputting a first drive signal to drive a fan to start operation, and at the same time, controlling a first timer to start timing, and when the timing of the first timer satisfies a first set duration, controlling the bipolar ion generation module to start working. Further disclosed is an air conditioner. According to the present invention, the usage effect of an air conditioner provided with a bipolar ion generation module is optimized in a convenient way.

## Description

### FIELD

The invention relates to the technical field of air conditioning, and in particular to a method for controlling air conditioner provided with bipolar ion generation module and an air conditioner.

### BACKGROUND

Bipolar ionization has a proven good effect for sterilizing or disinfecting, which has been widely used in fields including food processing, food packaging and food storage where requirements on air cleanliness are strict to provide a completely sterile environment. Reliable long-term performance of bipolar ionization, mainly relying on positive and negative ions generated, such as O₂₊ and O₂₋ produced under corona discharge having high chemical activity and being capable of reacting with water molecules in air to form H₂O₂ and the oxidant decomposes bacterial protein in a further chemical reaction process for sterilization, could prevent bacteria reproduces. A bipolar ion generator is of great assistance to inhibit the growth of microorganisms and bacteria in a closed environment.

The prior art is dedicated to applying a bipolar ion generator in air conditioning because its proven effect for sterilizing or disinfecting could improve indoor air quality, and additionally if outdoor air pollution gets worse air conditioners provided with a bipolar ion generator could serve as a purifier. Chinese patent CN207299229U discloses: "A bipolar ion generator set includes a base and an outer fixing plate; a through hole is formed at each of four corners of the base, four screw fixing seats are provided on an upper inner surface of the outer fixing plate; an air-conditioning hood plate is located between the base and the outer fixing plate and four screws respectively pass through the through holes on the four corners of the base to fixed on the four screw fixing seats on the inner surface of the outer fixing plate.".

From the prior art we know that a bipolar ion generator has been provided on an air conditioner, but how to operate it properly is not disclosed further. Due to the fact that a room where the an air conditioner is utilized is far larger than the size of a bipolar ion generator, if air flowing through the bipolar ion generator is not correctly adjusted or controlled, the concentration of positive and negative ions will rapidly decay in air ducts to a level failing to purify air.

### SUMMARY

In order to ensure the working effect of the bipolar ion generator, the present invention provides a method for controlling air conditioner provided with a bipolar ion generation module and an air conditioner.

The present invention provides a method for controlling air conditioner provided with a bipolar ion generation module, which includes the following steps: an air conditioner controller determines whether a purification mode control signal configured to initiate an operation of the bipolar ion generation module is received; the air conditioner controller determines whether an air conditioner is in a working state if the purification mode control signal is received; if the air conditioner is in the working state, initiating the bipolar ion generation module; if the air conditioner is in a standby state, outputting a first driving signal to drive a fan to start running, initiating a first timer and initiating the bipolar ion generation module if a counting time of the first timer meets a first set duration.

Further the method further comprises: turning on the air conditioner and enabling a second timer to start timing in the meanwhile; determining whether a counting time of the second timer is greater than or equal to the first set duration if the purification mode control signal is received; initiating the bipolar ion generation module by the air conditioner controller if the counting time of the second timer is greater than or equal to the first set duration; keeping waiting and initiating the bipolar ion generation module until the counting time of the second timer is equal to the first set duration if the counting time of the second timer is less than the first set duration.

Further the method further comprises: if when the purification mode control signal is received, the counting time of the second timer is greater than or equal to the first set duration, clearing the second timer; and enabling the second timer to restart timing when the bipolar ion generation module has turned off and the air conditioner keeps working; if when the purification mode control signal is received, the counting time of the second timer is less than the first set duration, keeping waiting until the counting time of the second timer is equal to the first set duration, clearing the second timer; and enabling the second timer to restart timing when the bipolar ion generation module has turned off and the air conditioner keeps working.

Further the method further comprises: if the air conditioner is in the working state and the bipolar ion generation module starts to work, the air conditioner controller determines whether a purification mode turn-off signal which is configured to terminate the operation of the bipolar ion generation module is received; if the purification mode turn-off signal is received, turning off the bipolar ion generation module and maintaining the air conditioner in the working state; if the air conditioner is in the working state and the bipolar ion generation module starts to work, the air conditioner controller determines whether a shutdown signal which is configured to turn off the air conditioner is received; if the shutdown signal is received, turning off the bipolar ion generation module and maintaining the air conditioner in the working state, starting a third timer at the same time and switching the air conditioner into the standby state if a counting time of the third timer reaches a second set duration.

Further the method further comprises: when the air conditioner is in the working state, the air conditioner monitors whether a fan failure signal is received; if the fan failure signal is received, outputting a disable signal to prohibit the bipolar ion generation module from working.

Further the method further comprises: the air conditioner controller further a first alarm signal at the same time as outputting the disable signal.

Further the method further comprises: when the bipolar ion generation module starts working, the air conditioner controller monitors whether a bipolar ion generation module fault signal is received; if the air conditioner controller receives the bipolar ion generation module fault signal, the air conditioner outputs a disable signal to prohibit the bipolar ion generation module from working and at the same time outputs a second alarm signal.

Preferably the first set duration and the second set duration are in a range from 1 to 15 seconds.

The control method disclosed in the present invention initiates the fan provided in the air conditioner in the first place, and then starts the first timer until the counting time of the first timer reaches to the first set duration, and then initiates the bipolar ion generation module; on one hand, it could enable the user to learn that the air conditioner responses immediately as receiving a command so as to avoid a situation that the user mistakenly believes that the air conditioner fails to respond or a wrong operation occurs so as to repeatedly turn on or turn off the air conditioner; on the other hand, the ion concentration in the air supplied to the air-conditioning room could reach to an expected level.

Another aspect of the present invention is to provide an air conditioner applying a method for controlling air conditioner provided with a bipolar ion generation module; the method includes the following steps: an air conditioner controller determines whether a purification mode control signal configured to initiate an operation of the bipolar ion generation module is received; the air conditioner controller determines whether an air conditioner is in a working state if the purification mode control signal is received; if the air conditioner is in the working state, initiating the bipolar ion generation module; if the air conditioner is in a standby state, outputting a first driving signal to drive a fan to start running, initiating a first timer and initiating the bipolar ion generation module if a counting time of the first timer meets a first set duration.

In the air conditioner disclosed in the present invention, the purification mode control signal is independent of the working state of the air conditioner. The air conditioner could fulfill air conditioning function and purification effect at the same time, but also could serve as a purifier independently, thereby improving the flexibility of the usage of air conditioner.

### BRIEF DESCRIPTION OF ACCOMPANYING DRAWINGS

In order to explain the embodiments of the present invention or the technical solutions in the prior art more clearly, the following will briefly introduce the drawings that need to be used accompanying with the description or the prior art. Obviously, the drawings in the following description are some embodiments of the present invention. For those of ordinary skill in the art, other drawings can be obtained based on these drawings without creative work.
Fig. 1 is a flowchart of a method for controlling air conditioner provided with a bipolar ion generation module according to a first aspect of the present invention;
Fig.2 is a flowchart of a method for controlling air conditioner provided with a bipolar ion generation module according to a second aspect of the present invention;
Fig.3 is a flowchart of a method for controlling air conditioner provided with a bipolar ion generation module according to a third aspect of the present invention;
Fig.4 is a flowchart of a method for controlling air conditioner provided with a bipolar ion generation module according to a fourth aspect of the present invention.

### DETAILED DESCRIPTION

To make objectives, technical solutions, and advantages of embodiments of the present invention better and more completely understood, the technical solutions in the embodiments of the present invention will be described clearly and completely in conjunction with accompanying drawings. Obviously, the described embodiments are a part of the embodiments of the present invention. Based on the embodiments of the present invention, all other embodiments obtained by those of ordinary skill in the art without creative work shall fall within the protection scope of the present invention.

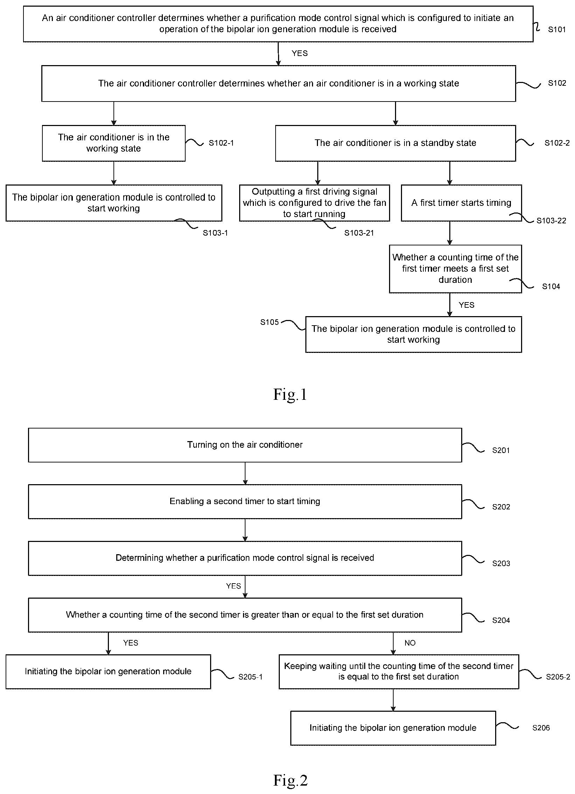

Fig.1 is a flowchart of a method for controlling air conditioner provided with bipolar ion generation module according to a first aspect of the present invention. As shown in Fig.1, the method is applied to an air conditioner provided with a bipolar ion generation module, the air conditioner generally refers to a split type with one outdoor unit installed in an open space and one indoor unit installed in an air-conditioned room; the air conditioner is further provided with an air conditioner controller which could be a microcontroller unit, namely a computer chip provided with a data processing module and a storage module comprising a read-only memory and a random memory, wherein the data processing module is configured to read programs configured to realize different control targets from the read-only memory, to process in the random access memory (RAM), and to cooperate with programs aiming for controlling user interface, sensors, as well as other input ports or output ports in the air conditioner. The bipolar ion generation module could selected from bipolar ion generation modules disclosed in the prior art, and how the bipolar ion generation module works for sterilization or purification is described in the background and is not repeated. It should be mentioned that the model name and parameters of both of the air conditioner controller and the bipolar ion generation module are not limited.

Referring to Fig.1, the method for controlling air conditioner provided with a bipolar ion generation module includes following steps.

Step S101: the air conditioner controller determines whether a purification mode control signal which is configured to initiate an operation of the bipolar ion generation module is received. The bipolar ion generation module is in connection with the air conditioner controller by a serial bus; the bipolar ion generation module and the air conditioner controller are in communication by means of single-channel half duplex asynchronous serial communication, and data group to be transmitted is configured in a preset data format in a form of binary code. The air conditioner controller preferably allocates an input port for the bipolar ion generation module only to receive an independent purification mode control signal which is configured to initiate an operation of the bipolar ion generation module. The purification mode control signal is generated by the user interaction interface which could be formed on one or more devices from a remote controller, a control panel, a wire controller, or mobile terminals capable of generating remote control signals including mobile phones, tablets, laptops, wearable devices, smart devices and the like.

Step S102: the air conditioner controller determines whether an air conditioner is in a working state.

In order to avoid a rapid decay in ion concentration, the air conditioner controller postpones the initiation of the bipolar ion generation module after receiving the independent purification mode control signal. The air conditioner controller firstly determines whether the air conditioner is in a working state; to be specific whether the air conditioner is in a working state could be obtained through data transmitted between a host unit and an auxiliary unit of the air conditioner. Further the working state indicates a state of the air conditioner when a process as follows ends, which includes the air conditioner receives a start-up command, and then the air conditioner controller provided in the host unit sends preset operating parameters and the start-up command to the auxiliary unit; the auxiliary unit receives the preset operating parameters and the start-up command to execute a protection process or an operation as programmed in a set mode. Preferably, a flag is preset for indicating that the air conditioner is in the working state in the program, which could be used by the air conditioner directly for determination: if the air conditioner controller could identify a valid flag value it could be determined that the air conditioner is in the working state. When the air conditioner is in the working state, a fan runs.

Step S102-1: the air conditioner controller determines that the air conditioner is in the working state. Step S103-1: the bipolar ion generation module is controlled to start working. Preferably, a windward side of the bipolar ion generation module is installed upstream of an air duct of the air conditioner and a leeward side of the bipolar ion generation module is installed downstream of the air duct of the air conditioner. When the air conditioner is working, the fan provided in the air duct of the air conditioner could guide air flowing towards an air outlet so the airflow in the air duct of the air conditioner could further lead ions generated by the bipolar ion generation module to flow from the windward side to the leeward side and quickly enter the air-conditioned room through the air outlet, which nearly avoids that the ions contact with other parts of the air outlet, thereby inhibiting the rapid decay of ion concentration and ensuring the effect for sterilization.

Step S102-2: if the air conditioner controller determines that the air conditioner is in a standby state. Then in Step S103-21: a first driving signal which is configured to drive the fan to start running is output. Further the standby state of the air conditioner indicates a state of the air conditioner when a process as follows ends, which includes: when the air conditioner is powered on, the host unit and the auxiliary unit will automatically communicate with each other in accordance with an established configuration and send a hardware running conditions of their own to the other end respectively in a form of a pulse sequence; after receiving information representing normal operation from the other end, the host unit and the auxiliary unit are respectively in a standby state and the air conditioner is in the standby state as well. In the Step S102-1 and the Step S102-2, the host unit could be either of the indoor unit or the outdoor unit, and the auxiliary unit is realized by the other one according to varied models and communication rules of the air conditioner, which are not limited here. The fan is arranged in the air duct of the air conditioner to guide air in the air duct to flow out from the air outlet. Simultaneously with the Step S103-21, in the Step S103-22 a first timer is controlled to start timing. The first timer may be either a built-in clock chip in the air conditioner controller, which is started by a software program, or a timing chip independent from the air conditioner controller.

Step S104: the air conditioner controller determines whether a counting time of the first timer meets a first set duration.

Step S105: if the counting time of the first timer meets the first set duration, the bipolar ion generation module is controlled to start working. It is because if the air conditioner is in the standby state an immediate initiation of the bipolar ion generation module as receiving the purification mode control signal will inevitably cause a large amount of ions generated by the bipolar ion generation module accumulating in the air duct and incapable of flowing. Aggravated by an uneven air density distribution in the air duct, ions generated will contact with inner wall of the air duct and neutralize and attenuate rapidly; hence the ion concentration in the output airflow will be far lower than an expected level capable of ensuring a good effect for sterilization and purification if the fan is turned on at this time. But by means of initiating the fan to run in the first place and then initiating the bipolar ion generation module as the counting time of the first timer meets the first set duration, on one hand it could enable the user to learn that the air conditioner responses immediately as receiving a command so as to avoid a situation that the user mistakenly believes that the air conditioner fails to respond or a wrong operation occurs so as to repeatedly turn on or turn off the air conditioner; on the other hand, the ion concentration in the air supplied to the air-conditioning room could reach to an expected level. With this method, the effect of the air conditioner with the bipolar ion generation module is optimized in a convenient way. The purification mode control signal is independent of the working state of the air conditioner. The air conditioner could have air conditioning and air purification effect at the same time, and also could be used as a purifier independently, so as to provide a more flexible way to use the air conditioner.

Fig.2 is a flowchart of a method for controlling an air conditioner provided with a bipolar ion generation module according to a second aspect of the present invention. As shown in Fig.2 the method includes:
Step S201, turning on the air conditioner: the air conditioner operates in a set mode in accordance with predetermined parameters.
Step S202, enabling a second timer to start timing by the air conditioner controller as the air conditioner is turned on; wherein the second timer could be a built-in clock chip in the air conditioner controller which could be started by a software program, or a timing chip independent of the air conditioner controller.
Step S203, determining whether a purification mode control signal is received by the air conditioner controller.
Step S204, determining whether a counting time of the second timer is greater than or equal to the first set duration if the purification mode control signal is received.
Step S205-1, initiating the bipolar ion generation module by the air conditioner controller if the counting time of the second timer is greater than or equal to the first set duration.
Step S205-2, keeping waiting until the counting time of the second timer is equal to the first set duration if the counting time of the second timer is less than the first set duration.
Step S206, initiating the bipolar ion generation module.

Provided with the method as shown in Fig.2, an air conditioner provided with a bipolar ion generation module could have an air conditioning function and a bipolar ion generation function independent of each other. The air conditioner could operate in a normal way and further to start the latter function to release ions when the initiation condition for the bipolar ion generation module is satisfied so as to ensure an expected ion concentration and improve purification effect.

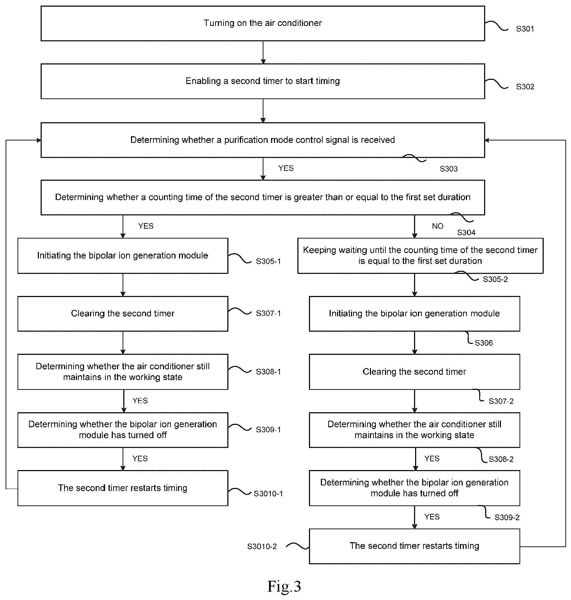

As shown in Fig. 3, the second timer is preferably controlled in a following manner includes:
Step S301, turning on the air conditioner;
Step S302, enabling a second timer to start timing;
Step S303, determining whether a purification mode control signal is received by the air conditioner controller;
Step S304, determining whether a counting time of the second timer is greater than or equal to the first set duration if the purification mode control signal is received;
Step S305-1, initiating the bipolar ion generation module if the counting time of the second timer is greater than or equal to the first set duration;
Step S305-2, keeping waiting until the counting time of the second timer is equal to the first set duration if the counting time of the second timer is less than the first set duration; in the process of waiting, the air conditioner keeps working in the set mode.
Step S306, initiating the bipolar ion generation module when the counting time of the second timer is equal to the first set duration.
Step S307-1, clearing the second timer as initiating the bipolar ion generation if the counting time of the second timer is greater than or equal to the first set duration when the purification mode control signal is received. The fine control on the second timer is designed for a situation that in hot summer or cold winter users prefers to continuously use the air conditioner for a long time to maintain the temperature in the air-conditioned room within a comfortable range, but in fact when the air conditioner is working there may be no one in the air-conditioned room. However, the sterilization and purification of the air is only necessary when someone in the air conditioned room or at a certain time period. For example, the air conditioner may be kept running continuously for 24 hours, and the purification function is only necessary to be turned on twice during certain periods, such as early morning and evening, a step to clear the second timer properly could meet the control requirement for this situation.

To be specific, after the second timer is cleared, as shown in Step S308-1, the air conditioner controller will determine whether the air conditioner still maintains in the working state. If the air conditioner has switched to the standby state, the second timer maintains in a reset state because it means that the air conditioner is malfunctioning probably; if the air conditioner has maintained in the working state, then in Step S309-1, the air conditioner controller determines whether the bipolar ion generation module has turned off; then in Step S3010-1, if it is determined that the bipolar ion generation module has turned off and the air conditioner keeps working, the second timer restarts timing; and further cyclically determining whether the purification mode control signal is received and determining whether a counting time of the second timer is greater than or equal to the first set duration.

Step S307-2, keeping waiting until the counting time of the second timer is equal to the first set duration if the counting time of the second timer is less than the first set duration when the purification mode control signal is received; then initiating the bipolar ion generation module and clearing the second timer at the same time to satisfy the requirement of fine control.

To be specific, after the second timer is cleared, as shown in Step S308-2, the air conditioner controller will determine whether the air conditioner still maintains in the working state. If the air conditioner has switched to the standby state, the second timer maintains in a reset state; if the air conditioner has maintained in the working state, then in Step S309-2, the air conditioner controller determines whether the bipolar ion generation module has turned off; then in Step S3010-2, if it is determined that the bipolar ion generation module has turned off and the air conditioner keeps working, the second timer restarts timing; and further cyclically determining whether the purification mode control signal is received and determining whether a counting time of the second timer is greater than or equal to the first set duration.

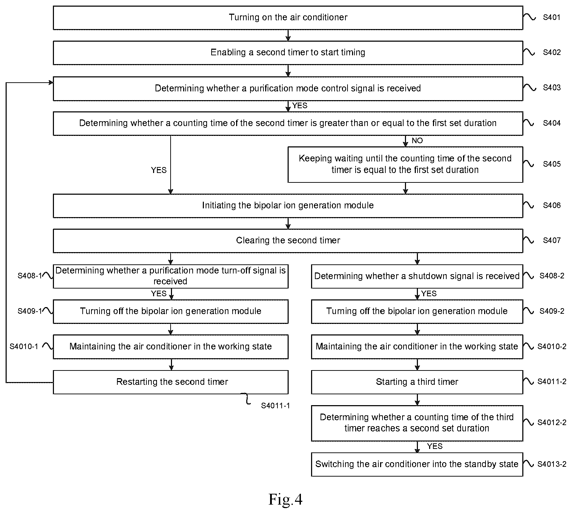

More specifically whether the bipolar ion generation module has turned off could be determined by checking whether a purification mode turn-off signal is received. As shown in Fig.4, after clearing the second timer the following steps are further included:
Step S408-1, determining whether a purification mode turn-off signal is received by the air conditioner controller;
Step S409-1, turning off the bipolar ion generation module by the air conditioner controller if the purification mode turn-off signal is received.
Step S4010-1, maintaining the air conditioner in the working state.
Step S4011-1, restarting the second timer by the air conditioner controller.

Alternatively the air conditioner may also receive a shutdown signal sent by users through an interactive interface, shown in Fig.4, the control method further includes:
Step S408-2, determining whether a shutdown signal is received by the air conditioner controller;
Step S409-2, turning off the bipolar ion generation module firstly by the air conditioner controller if the shutdown signal is received;
Step S4010-2, maintaining the air conditioner in the working state by the air conditioner controller;
Step S4011-2, starting a third timer by the air conditioner controller at the same time;
Step S4012-2, determining whether a counting time of the third timer reaches a second set duration by the air conditioner controller;
Step S4013-2, switching the air conditioner into the standby state if the counting time of the third timer reaches the second set duration;

The first set duration and the second set duration are preferably in a range from 1 to 15 seconds and could be adjusted in accordance with different types and capacities of air conditioner and will not be limited further.

In the above control process, after the air conditioner controller receives the shutdown signal, it is preferably to firstly turn off the bipolar ion generation module because at this time some ions have already generated and remained in the air duct which could be blown into the air-conditioned room by the fan which is postponed to be switched off so as to ensure an optimal purification and sterilization effect.

Preferably when the air conditioner is in the working state, the air conditioner controller keeps monitoring whether a fan failure signal is received. If the air conditioner controller receives the fan failure signal, it will output a disable signal, to firstly control the bipolar ion generation module to stop working and prohibit the bipolar ion generation module from working. Accordingly the bipolar ion generation module could be protected to avoid invalid operation so as to prolong its service life. The disabling operation is fully automatic so as to reduce manual maintenance workload. The air conditioner controller may further output a first alarm signal while outputting the disable signal. The first alarm signal may be a sound alarm signal or a signal light, which is not limited further.

Similarly when the air conditioner controller initiates the bipolar ion generation module, the air conditioner controller also keeps monitoring whether a bipolar ion generation module fault signal is received. If the air conditioner controller receives the bipolar ion generation module fault signal, the air conditioner outputs a disable signal to prohibit the bipolar ion generation module from working and at the same time outputs a second alarm signal.

The present invention also discloses an air conditioner, which adopts the methods for controlling an air conditioner provided with a bipolar ion generation module disclosed in any of the above-mentioned embodiments. The steps methods for controlling an air conditioner provided with a bipolar ion generation module could be referred to the detailed description of the above-mentioned embodiments, and the accompanying drawings, which will not be repeated further. The air conditioner adopting the above control method can achieve the same technical effect.

Finally, it should be noted that the above embodiments are only used to illustrate the technical solutions of the present invention, not to limit it; although the present invention has been described in detail with reference to the foregoing embodiments, those of ordinary skill in the art should understand that: the technical solutions recorded in the foregoing embodiments could be modified, or some of the technical features could be equivalently replaced; these modifications or replacements do not cause the essence of the corresponding technical solutions to deviate from the spirit and scope of the technical solutions of the embodiments of the present invention.

## Claims

1. A method for controlling air conditioner provided with a bipolar ion generation module, **characterized in that** the method comprises the following steps:
an air conditioner controller determines whether a purification mode control signal configured to initiate an operation of the bipolar ion generation module is received;
the air conditioner controller determines whether an air conditioner is in a working state if the purification mode control signal is received;
if the air conditioner is in the working state, initiating the bipolar ion generation module;
if the air conditioner is in a standby state, outputting a first driving signal to drive a fan to start running, initiating a first timer and initiating the bipolar ion generation module if a counting time of the first timer meets a first set duration.

2. The method for controlling air conditioner provided with a bipolar ion generation module according to claim 1, **characterized in that** the method further comprises:
turning on the air conditioner and enabling a second timer to start timing in the meanwhile;
determining whether a counting time of the second timer is greater than or equal to the first set duration if the purification mode control signal is received;
initiating the bipolar ion generation module by the air conditioner controller if the counting time of the second timer is greater than or equal to the first set duration;
keeping waiting and initiating the bipolar ion generation module until the counting time of the second timer is equal to the first set duration if the counting time of the second timer is less than the first set duration.

3. The method for controlling air conditioner provided with a bipolar ion generation module according to claim 2, **characterized in that**, the method further comprises:
if when the purification mode control signal is received, the counting time of the second timer is greater than or equal to the first set duration, clearing the second timer; and enabling the second timer to restart timing when the bipolar ion generation module has turned off and the air conditioner keeps working;
if when the purification mode control signal is received, the counting time of the second timer is less than the first set duration, keeping waiting until the counting time of the second timer is equal to the first set duration, clearing the second timer; and enabling the second timer to restart timing when the bipolar ion generation module has turned off and the air conditioner keeps working.

4. The method for controlling air conditioner provided with a bipolar ion generation module according to claim 3, **characterized in that**, the method further comprises:
if the air conditioner is in the working state and the bipolar ion generation module starts to work, the air conditioner controller determines whether a purification mode turn-off signal which is configured to terminate the operation of the bipolar ion generation module is received; if the purification mode turn-off signal is received, turning off the bipolar ion generation module and maintaining the air conditioner in the working state;
if the air conditioner is in the working state and the bipolar ion generation module starts to work, the air conditioner controller determines whether a shutdown signal which is configured to turn off the air conditioner is received; if the shutdown signal is received, turning off the bipolar ion generation module and maintaining the air conditioner in the working state, starting a third timer at the same time and switching the air conditioner into the standby state if a counting time of the third timer reaches a second set duration.

5. The method for controlling air conditioner provided with a bipolar ion generation module according to claim 4, **characterized in that**, the method further comprises:
when the air conditioner is in the working state, the air conditioner monitors whether a fan failure signal is received; if the fan failure signal is received, outputting a disable signal to prohibit the bipolar ion generation module from working.

6. The method for controlling air conditioner provided with a bipolar ion generation module according to claim 5, **characterized in that**, the method further comprises:
the air conditioner controller further a first alarm signal at the same time as outputting the disable signal.

7. The method for controlling air conditioner provided with a bipolar ion generation module according to claim 6, **characterized in that**, the method further comprises:
when the bipolar ion generation module starts working, the air conditioner controller monitors whether a bipolar ion generation module fault signal is received; if the air conditioner controller receives the bipolar ion generation module fault signal, the air conditioner outputs a disable signal to prohibit the bipolar ion generation module from working and at the same time outputs a second alarm signal.

8. The method for controlling air conditioner provided with a bipolar ion generation module according to any one of claim 1 to 7, **characterized in that**, the first set duration and the second set duration are in a range from 1 to 15 seconds.

9. An air conditioner, **characterized in that**, the air conditioner applies to the for controlling air conditioner provided with a bipolar ion generation module according to any one of claim 1 to 8.
